(19) Europäisches Patentamt / European Patent Office / Office européen des brevets

(11) **EP 2 108 268 A1**

(12) **DEMANDE DE BREVET EUROPEEN**

(43) Date de publication:
**14.10.2009 Bulletin 2009/42**

(21) Numéro de dépôt: **09157300.6**

(22) Date de dépôt: **03.04.2009**

(51) Int Cl.:
*A23L 1/29* (2006.01)  *A23L 2/00* (2006.01)
*A61K 36/45* (2006.01)  *A61P 13/00* (2006.01)
*A61P 31/04* (2006.01)

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **09.04.2008 FR 0852386**
**27.06.2008 US 215533**

(71) Demandeur: **Diana Naturals**
**35560 Antrain (FR)**

(72) Inventeurs:
• **Besnard, Mathieu**
**35340, LIFFRE (FR)**
• **Inisan, Claude**
**35830, BETTON (FR)**
• **Rousseau, Isabelle**
**35460, SELLE-EN-COGLES (FR)**

(74) Mandataire: **Zapalowicz, Francis et al**
**Bureau Casalonga & Josse**
**Bayerstrasse 71/73**
**80335 München (DE)**

(54) **Extrait de canneberge d'Amérique et son utilisation**

(57) La présente invention concerne un extrait issu de Vaccinium ainsi que le procédé d'obtention de cet extrait. Cet extrait riche en proanthocyanidines est utilisable en tant que préparation alimentaire ou neutraceutique.

EP 2 108 268 A1

**Description**

La présente invention concerne un extrait issu de *Vaccinium* ainsi que le procédé d'obtention de cet extrait. Cet extrait riche en proanthocyanidines est utilisable en tant que préparation alimentaire ou nutraceutique.

*Vaccinium macrocarpon* Aiton (canneberge d'Amérique ou en anglais American cranberry) est un arbuste de petite taille plus ou moins rampant et ne dépassant pas 30 cm qui croît spontanément uniquement dans l'Est de l'Amérique du Nord depuis les Carolines jusqu'au Canada. Il prospère sur sols acides pauvres et marécageux, parmi des tourbières, landes, ou dans les forêts de conifères. Le fruit est une petite baie mesurant 10 à 20 mm de diamètre. A maturité, sa coloration est rouge vif et sa saveur acidulée et astringente.

La baie de canneberge d'Amérique est couramment consommée en alimentation en Amérique du Nord depuis plusieurs centaines d'années. Elle ne contient pas de constituants en quantités suspectes connus pour leur toxicité et il n'a pas été rapporté d'incident suite à sa consommation. Aux doses habituelles, les effets indésirables sont rares. Les troubles digestifs constituent la principale cause d'effets délétères mentionnés lors d'études cliniques. Ils surviennent lorsque la consommation de jus devient très excessive (3-4 litres/jour) et se traduisent surtout par des diarrhées et des douleurs gastro-intestinales (Blumenthal M., & al., The ABC clinical guide to herbs, American Botanical Council, Austin, Texas, 2003, pp. 73-83).

La baie de canneberge d'Amérique, se caractérise par sa richesse en flavonoïdes et tout particulièrement en oligomères proanthocyanidiques de type A, famille de composés rarement rencontrés dans les végétaux alimentaires.

Les proanthocyanidines ou tanins condensés sont des composés phénoliques polymériques d'unités flavanol, telles que la catéchine, l'épicatéchine et leurs dérivés.

Les premiers éléments rapportés à propos des usages thérapeutiques de la baie remonteraient au 17ème siècle. Les amérindiens semblent l'avoir appliquée sous forme de cataplasmes sur les plaies. Par la suite, les navigateurs et les colons l'employèrent en prévention du scorbut et pour traiter des symptômes variés : troubles circulatoires et hépatiques, douleurs stomacales, fièvres ... (Blumenthal et al., 2003). L'usage de la canneberge d'Amérique dans le traitement des infections des voies urinaires est évoqué dans des articles parus aux Etats-Unis il y a plus de cent ans.

Il fut découvert en 1984 que du jus de canneberge d'Amérique administré par voie orale à la souris pendant 14 jours, génère des urines capables d'inhiber fortement l'adhérence de souches uropathogènes d'*Escherichia coli* à des cellules uroépithéliales (Sobota A.E., Inhibition of bacterial adherence by cranberry juice: potential use for the treatment of urinary tract infections., J. Urol., 1984, 131, 5, 1013-6).

Des fractions concentrées en proanthocyanidines d'autres espèces du genre *Vaccinium,* notamment V. *myrtillus* (Myrtille), posséderaient des activités similaires (Howell A. B., & al., Inhibition of the adherence of P-fimbriated Escherichia coli to uroepithelial-cell surfaces by proanthocyanidins extracts from cranberries, New Eng. J. Med., 1998, 339, 15, 1085; Ofek L. & al., Anti-Escherichia coli adhesion activity of cranberry and blueberry juices., New Eng. J. Med., 1991, 324, 22, 1599).

Les capacités d'extraits de baies de *Vaccinium macrocarpon* enrichis en proanthocyanidines à inhiber l'adhérence de souches d'*E. coli* uropathogènes sur des cellules uroépithéliales ont été constatées *in vitro* à des concentrations comprises entre 10 et 50 $\mu$g/ml (Howell et al., 1998).

Lors d'une étude entreprise au Canada, la forme comprimée contenant du jus déshydraté de canneberge américaine fut considérée comme préférable au jus de canneberge en raison d'un meilleur suivi de traitement à son terme, le jus ayant une saveur peu appréciée par certains sujets (Stothers L., A. randomised trial to evaluate effectiveness and cost effectiveness of naturopathic cranberry products as prophylaxis against urinary tract infection in women., Can. J. Urol., 2002, 9, 3, 1558-62).

Cependant, le faible dosage de ces comprimés en proanthocyanidines obligeaient les patients à ingérer plusieurs comprimés par jour, rendant fastidieux ce traitement.

Dans son avis 2003-SA-0352 du 06 avril 2004, l'Agence Française de Sécurité Sanitaire des Aliments (AFSSA) a approuvé l'allégation suivante : « Le jus concentré ou la poudre de jus de fruits de *Vaccinium macrocarpon* contribue à diminuer la fixation de certaines bactéries *Escherichia coli* sur les parois des voies urinaires. L'AFSSA considère ainsi que l'allégation « contribue à diminuer la fixation de certaines bactéries *E. coli* sur les parois des voies urinaires » peut être acceptée pour de la canneberge fraîche/congelée et purée de canneberge sous réserve d'une consommation journalière de quantités du produit apportant au minimum 36 mg de proanthocyanidines (AFSSA, 2007).

Ainsi, il subsiste un besoin de compositions à base de canneberge, riche en proanthocyanidines, faciles à absorber par un être humain en dose journalière sans que les contraintes du traitement en terme de goût du produit à ingérer ou en nombre de comprimés à avaler ne pèsent trop sur ce patient, rendant son suivi journalier aisé.

La demanderesse a mis au point de nouveaux extraits de *Vaccinium,* qui constituent l'objet de l'invention.

L'invention a également pour objet le procédé d'obtention de cette fraction.

Un autre objet est constitué par l'application de cet extrait comme complément alimentaire ou nutraceutique.

D'autres objets apparaîtront à la lecture de la description et des exemples qui suivent.

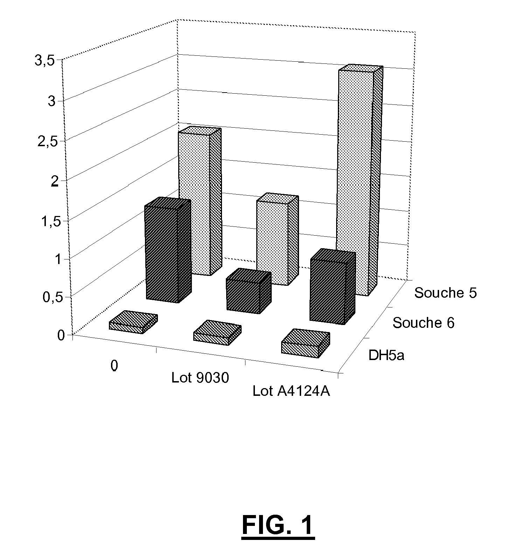

La figure 1 montre la quantité de bactéries fixées sur la plaque exprimée en densité optique en fonction de l'extrait de canneberge testé (0 = sans extrait, lot 9030 = extrait AFCANN 9030 de Diana Naturals, lot A4124A = extrait du commerce) et de la bactérie utilisée (souche DH5$\alpha$ = souche de référence, souches 5 et 6 = souches uropathogènes).

L'extrait conforme à l'invention est issu de végétaux du genre *Vaccinium,* comprenant au moins 15% en poids de proanthocyanidines exprimées en équivalent procyanidine Cl par rapport au poids sec de l'extrait.

De préférence, le vaccinium est *Vaccinium macrocarpon* ou canneberge d'Amérique. Cet extrait provient préférentiellement de la baie de ce végétal.

De préférence, cet extrait comporte plus de 5% de sucre provenant du fruit par rapport au poids sec de l'extrait.

De préférence, cet extrait présente un degré de polymérisation moyen des proanthocyanidines (c'est-à-dire une longueur moyenne de la chaîne des tanins) supérieur à 5. Plus préférentiellement, ce degré est supérieur à 6. Encore plus préférentiellement, le degré de polymérisation moyen des proanthocyandines dans l'extrait de l'invention est supérieur à 8.

De préférence, cet extrait de canneberge conserve une quantité d'anthocyanes identiques à celle du jus de canneberge.

Le procédé d'obtention de l'extrait riche en proanthocyanidines selon l'invention ne modifiant pas les proportions des anthocyanes de la canneberge, cette caractéristique de l'extrait de canneberge reste donc proche de celles du jus avant traitement.

Le procédé d'obtention d'un extrait selon l'invention est caractérisé par le fait que l'on procède à une mise en solution aqueuse du fruit de la canneberge d'Amérique, que l'on procède à une séparation solide-liquide, que l'on ajoute un solvant organique puis que l'on procède à une déshydratation du produit obtenu pour obtenir l'extrait selon l'invention.

De préférence, le pH de la solution aqueuse est compris entre 2 et 6, la température est inférieure à 95 °C et la pression est comprise entre 1 et 3 atmosphères.

De façon optionnelle, du sulfite ou du disulfite est ajouté au cours de la phase d'extraction aqueuse à des concentrations comprises entre 0,01 et 1% du volume de la solution aqueuse, et de préférence à une concentration de 0.3%. Du sulfite de sodium, de calcium, de potassium, du disulfite de sodium ou potassium peuvent être utilisés.

Par son action antioxydante et antiseptique, le sulfite ou disulfite contribue à préserver l'activité de l'extrait selon l'invention au cours du procédé d'obtention.

De préférence, la séparation solide-liquide s'effectue à un pH compris entre 2 et 7, à une température comprise entre 15 et 70 °C et à une pression comprise entre 1 et 15 atmosphères.

De préférence, le solvant organique est ajouté dans des proportions de 0 à 15 en volume par rapport au volume de la canneberge en solution aqueuse.

De préférence, le solvant organique utilisé est un alcool, un aldéhyde, ou un ester.

Plus préférentiellement, le solvant organique ajouté est l'éthanol, l'acétaldéhyde ou l'acétate d'éthyle.

La déshydratation du produit obtenu se fait de préférence sous pression réduite c'est-à-dire inférieure à 1 atmosphère, à une température inférieure à 60 °C.

De façon optionnelle, une étape de déshydratation finale est effectuée à une température comprise entre 80 et 190 °C et une pression comprise entre 0,5 et 2 atmosphères.

L'extrait de l'invention est obtenu par le procédé précédemment décrit.

Cet extrait de canneberge d'Amérique possédant les caractéristiques énoncées, susceptible d'être obtenu selon le procédé précédemment décrit, est utilisable en tant que complément alimentaire ou nutraceutique.

L'extrait riche en proanthocyanidines selon l'invention a la particularité de prévenir l'adhérence des bactéries, et plus particulièrement des bactéries *E. coli* à la surface des parois du tractus urinaire.

L'invention a également pour objet une composition comprenant, entre autres, l'extrait riche en proanthocyanidines précédemment décrit.

Les compositions selon l'invention peuvent être ingérées. Selon le mode d'administration, la composition selon l'invention peut se présenter sous toutes les formes habituellement utilisées en alimentaire ou nutraceutique.

Les compositions selon l'invention peuvent notamment se présenter sous forme de gélules, de comprimés, de poudre ou de boissons.

Les compositions nutraceutiques ou alimentaires de la présente invention sont formulées classiquement selon les applications auxquelles elles sont destinées.

Les exemples suivants illustrent l'invention sans la limiter aucunement.

Exemple 1 : Procédé d'obtention de l'extrait AFCANN9030 selon l'invention

Des canneberges d'amérique ont été mises en solution en phase aqueuse à raison de 1 volume de baies pour 5 volumes d'eau. Après broyage et séparation solide-liquide par décantation à une température de 25°C, on ajoute de l'acétate d'éthyle à raison de 5 volumes d'acétate pour un volume de canneberge en phase aqueuse, à une température de 20°C, un pH de 4.5, sous une pression de 1 atm, puis on sépare les deux phases obtenues. On effectue ensuite une déshydratation sous une pression réduite de 0,1 à 0,5 atm, à une température de 30 à 40°C, puis une seconde déshydratation à une pression de 0,9 à 1,5 atm, et une température de 145°C.

Le produit AFCANN9030 obtenu est une poudre pourpre.

Exemple 2 : Dosage des proanthocyanidines dans l'extrait AFCANN9030 obtenu

La méthode de Bate-Smith modifiée par Porter pour le dosage des proanthocyanidines a été utilisée (Bate-Smith E. C. Phytochemistry, 1973, 12, pp907-912 et Porter. L. J., & al., Phytochemistry, 1986, 25, 223-230).

La réaction de dosage se fait en deux étapes. Dans un premier temps, les polymères de flavan-3-ol sont hydrolysés dans un milieu butanol-HCl. L'hydrolyse est totale et libère des monomères de type catéchine et épicatéchine. La deuxième étape du dosage consiste à oxyder ces monomères sous l'action de $Fe^{III}$. Le produit de l'oxydation est la cyanidine et est quantifié à l'aide d'un spectrophotomètre à 541 nm.

On prépare le réactif de Bate-Smith en mélangeant 500 mL de HCl à la concentration de 35 %, 500 mL de n-butanol et 150 mg de $Fe_2(SO_4)_3$.

On dissout 100 mg d'extrait de canneberge d'Amérique dans 100 mL d'un mélange d'eau déminéralisée et d'éthanol 1/1 en poids. On mélange 2 mL de cette solution avec 6 mL de réactif de Bate-Smith dans un premier tube. On transfère la moitié de la solution dans un second tube. On laisse le premier tube à l'obscurité pendant que le second tube est chauffé 30 minutes dans un bain-marie à la température de 100 °C puis laissé dans l'obscurité pendant 10 minutes. La mesure des absorbances (DO) des deux tubes est effectuée à 541 nm en faisant un blanc avec de l'eau déionisée.

Les résultats sont exprimés en équivalent proanthocyanidine C1 selon la formule suivante.

$$\% \text{ proanthocyanidines} = \frac{([DO541 \text{ tube } 2 - DO \ 541 \text{ tube } 1] - 0.0183) \times 100}{5{,}1407 \times \text{concentration de l'extrait en g/L}}$$

La mesure de la concentration en proanthocyanidines de l'extrait AFCANN9030 est de 30 % $\pm$ 2 exprimée en équivalent proanthocyanidine Cl.

Il est à noter que la quantification des proanthocyanidines tient compte des anthocyanes initialement présentes dans le milieu et l'absorption due à ces anthocyanes est soustraite de l'absorption lue après la réaction de Porter.

Exemple 3 : Caractérisation de l'extrait de l'exemple 1 (AFCANN9030)

|  | Extrait de l'exemple 1 |
|---|---|
| % de proanthocyanidines eq procyanidine C1 | 30,5 % |
| % PPT eq catéchine (UV 280nm) | 17,3 % |
| % Flavonols eq rutine (UV 354nm) | 2,4 % |
| % Flavonols eq quercétine (UV 354nm) | 1,4 % |
| Fructose (lot 3808001) | 24,4 mg/g |
| Glucose (lot 3808001) | 110,8 mg/g |
| Sucrose (lot 3808001) | ND |
| Résidu sec total | 97,5 % |
| Protéine (N*6,25) Dumas | 2,7 % |
| Mat. Grasse par hydrolyse | <1 % |
| Mat. Minérales | 2,4 % |

(suite)

|  | Extrait de l'exemple 1 |
|---|---|
| Fibres alimentaires totales | 13,5 % |
| eq : équivalent<br>PPT : polyphénols totaux | |

Exemple 4 : Composition de l'extrait obtenu à l'exemple 1 (AFCANN9030)

La thioacidolyse est une autre technique permettant de déterminer la teneur en proanthocyanidines ainsi que le degré de polymérisation moyen des proanthocyanidines (Guyot et al., J.Agric.Food Chem., 1998, 1698-1705).

Cette technique est également basée sur l'hydrolyse des polymères proanthocyanidoliques en milieu acide (HCl). L'$\alpha$-toluènethiol présent dans le milieu réactionnel réagit avec les monomères libérés lors de l'hydrolyse et les composés formés sont quantifiés en chromatographie liquide (HPLC).

Résultats d'analyses après thioacidolyse

|  | Extrait de l'exemple 1 |
|---|---|
| DPm | 8.5 |
| Teneur tanins mg/g poudre | 191.5 |
| %epi | 71.5 |
| %cat | 0.9 |
| %dimA | 27.6 |
| DPm:degré de polymérisation moyen (longueur moyenne de la chaîne des tanins) :<br>%epi = % épicatéchine<br>%cat = % catéchine<br>%dimA = % dimère de type A | |

Exemple 5 : test d'adhésion de l'extrait selon l'invention sur biofilm

La souche bactérienne DH5$\alpha$ qui ne possède pas de fimbriae est utilisée dans ce test comme témoin négatif d'adhésion. Les souches bactériennes 5 et 6 sont des souches de *E.coli* uropathogènes provenant d'isolats cliniques.

Les souches sont cultivées dans du milieu CFA à 37°C. La composition de ce milieu de culture est la suivante : dans 1 litre d'eau déionisée, on ajoute 10 grammes de casamino acides (Difco), 1,5g d'extrait de levure, 0,05g de MgSO$_4$ et 0,005g de MnCl$_2$.

Pour chaque souche bactérienne , on teste l'adhésion de la souche seule, ou en présence soit de canneberge du lot AFCANN9030 selon l'invention (Diana Naturals), contenant 30% de proanthocyanidines, soit de canneberge du lot A4124A (Echantillon commercial) contenant 3% de proanthocyanidines. Les échantillons de canneberge sont élaborés afin d'obtenir une concentration en proanthocyanidines de 500$\mu$g/mL, soit une dilution de 16,7 mg du lot 9030 ou de 167 mg du lot A4124A dans 10 mL d'eau. Ces solutions sont ensuite diluées au 1/10ème dans de l'eau pour effectuer les tests.

Une culture de nuit de la bactérie à étudier est utilisée, et 0,2 mL de cette culture est déposé dans une microplaque de 96 puits (Greiner) avec ou sans l'échantillon de canneberge à tester, dilué à nouveau au 1/5ème dans de l'eau. Chaque test est effectué en triplicata. Comme témoin négatif de culture, on dépose du milieu CFA non ensemencé avec l'un ou l'autre des échantillons de canneberge à tester.

Cette microplaque est placée à l'incubateur à 35°C pendant 24h.

Le lendemain, après élimination du milieu de culture, et lavage des puits à l'eau distillée, on ajoute dans chaque puits 200 $\mu$L de méthanol pendant 15 min. Apres élimination du méthanol, on sèche la plaque à l'étuve pendant 30 min. Puis, on ajoute 200 $\mu$L de cristal violet (Sigma) pendant 10 min. Après élimination du colorant et lavage des puits à l'eau distillée, on ajoute 200 $\mu$L de solution acide acétique/éthanol (vol/vol) pour détacher le biofilm produit. La lecture s'effectue avec un spectrophotomètre à une longueur d'onde de 570nm. Plus la valeur de la densité optique est élevée, plus le nombre de bactéries fixé sur la plaque est important.

Les résultats de la figure 1 montrent que l'extrait selon l'invention testé présente une densité optique inférieure à celle de l'extrait A4124A, donc une adhésion des bactéries sur le puits de plaque inférieure à celle du lot A4124A quelque soit la bactérie testée. Or, la concentration en proanthocyanidines présente dans le milieu de culture est la même dans le test avec l'extrait selon l'invention et le test avec l'extrait A4124A. L'effet d'inhibition de l'adhésion des bactéries à la microplaque attribué aux proanthocyanidines présentes dans le milieu à des concentrations équivalentes est donc plus important pour l'extrait selon l'invention que pour l'extrait A4124A, alors même que les concentrations en proanthocyanidines sont équivalentes. Ainsi, les proanthocyanidines présentes dans l'extrait selon l'invention ont un effet d'inhibition de l'adhésion des bactéries plus important que l'extrait A4124A à la même concentration massique.

Il est connu que cet extrait commercialisé (Lot A4124A) présente un degré de polymérisation moyen des proanthocyanidines (c'est-à-dire une longueur moyenne de la chaîne des tanins) de l'ordre de 3 à 4. Par ailleurs, l'extrait selon l'invention présente un degré moyen de polymérisation des proanthocyanidines de 8,1.

Il est donc possible de déduire de ces résultats que le degré de polymérisation moyen des proanthocyanidines dans l'extrait de canneberge selon l'invention joue un rôle sur l'adhésion des bactéries uropathogènes sur la surface du puits. Ainsi, l'extrait selon l'invention a une efficacité antiadhésive accrue par rapport au produit commercial A4124A.

Exemple 6 : exemple de formulation d'une gélule

Pour la fabrication d'une gélule de 250 mg au total, on mélange

- 240 mg d'extrait selon l'invention titré à 15 % de proanthocyanidines exprimées en équivalent procyanidine C1 par rapport au poids sec de l'extrait.
- 10 mg de cellulose microcristalline

Chaque gélule contient 36 mg de proanthocyanidines.

Exemple 7 : exemple de formulation d'une gélule

Pour la fabrication d'une gélule de 300 mg au total, on mélange

- 200 mg d'extrait AFCANN9030 (titrée à 30,5 % de proanthocyanidines exprimées en équivalent procyanidine C 1 par rapport au poids sec de l'extrait).
- 100 mg de cellulose microcristalline

Chaque gélule contient 61 mg de proanthocyanidines.

Exemple 8 : exemple d'une boisson selon l'invention

On prépare 100 grammes de la composition suivante :

|  | Quantité en grammes |
|---|---|
| Extrait AFCANN9030 | 0.68 |
| Poudre de jus clair de framboise (vendu sous la dénomination AFFRAM0013 chez Diana Naturals) | 56,3 |
| Poudre de jus clair de fraise (vendu sous la dénomination AFFRAI0013 chez Diana Naturals) | 29,3 |
| Poudre de jus de cassis (vendu sous la dénomination AFCASS9003 chez Diana Naturals) | 11,3 |
| Sucralose | 0,23 |
| Acide citrique | 2,25 |

Pour préparer la boisson, on mélange ensuite 22 grammes de cette composition en poudre dans un volume final de 500ml d'eau. Après agitation, une boisson au goût prononcé de framboise est prête à être consommée.

Une ration journalière de 500 mL de cette boisson apporte 45 milligrammes de proanthocyanidines.

**Revendications**

1. Extrait issu de végétaux du genre *vaccinium*, **caractérisé par le fait qu'**il comprend au moins 15% en poids de proanthocyanidines exprimées en équivalent procyanidine C1 par rapport au poids sec de l'extrait.

2. Extrait selon la revendication 1, dans lequel le végétal du genre vaccinium est *vaccinium macrocarpon* ou canneberge d'Amérique.

3. Extrait selon l'une quelconque des revendications 1 ou 2, **caractérisé par le fait que** l'on utilise la baie de la canneberge d'Amérique.

4. Extrait selon l'une quelconque des revendications 1 à 3, **caractérisé en ce que** le degré de polymérisation moyen des proanthocyanidines est supérieur à 5.

5. Extrait selon la revendication 4, **caractérisé en ce que** le degré de polymérisation moyen des proanthocyanidines est supérieur à 6.

6. Extrait selon la revendication 5, **caractérisé en ce que** le degré de polymérisation moyen des proanthocyanidines est supérieur à 8.

7. Procédé de préparation d'un extrait selon l'une quelconque des revendications 1 à 6, **caractérisé par le fait que** l'on procède à une mise en solution aqueuse de la canneberge d'Amérique, que l'on procède à une séparation solide-liquide, que l'on ajoute un solvant organique puis que l'on procède à une déshydratation du produit obtenu pour obtenir l'extrait.

8. Procédé de préparation d'un extrait selon la revendication 7, dans lequel on procède à une seconde déshydratation.

9. Procédé de préparation d'un extrait selon l'une des revendications 7 ou 8, **caractérisé par le fait que** le solvant organique est un alcool.

10. Procédé de préparation d'un extrait selon la revendication 9, dans laquelle l'alcool est l'éthanol.

11. Procédé de préparation d'un extrait selon l'une des revendications 7 ou 8, **caractérisé par le fait que** le solvant organique est un aldéhyde.

12. Procédé de préparation d'un extrait selon la revendication 11, dans laquelle l'aldéhyde est l'acétaldehyde.

13. Procédé de préparation d'un extrait selon l'une des revendications 7 ou 8, **caractérisé par le fait que** le solvant organique est un ester.

14. Procédé de préparation d'un extrait selon la revendication 13, dans laquelle l'ester est l'acétate d'éthyle.

15. Procédé selon l'une quelconque des revendications 7 à 14, **caractérisé par le fait que** le solvant est ajouté dans des proportions de 0 à 15 en volume par rapport au volume de la canneberge en solution aqueuse.

16. Extrait selon l'une quelconque des revendications 1 à 6, **caractérisé par le fait qu'**il est susceptible d'être obtenu par le procédé tel que défini dans l'une quelconque des revendications 7 à 14.

17. Composition alimentaire ou nutraceutique, **caractérisé par le fait qu'**elle contient un extrait tel que défini dans l'une quelconque des revendications 1 à 6 et 16.

18. Utilisation de l'extrait tel que défini dans l'une quelconque des revendications 1 à 6 et 16 pour contribuer à diminuer la fixation de certaines bactéries *E. coli* sur les parois des voies urinaires.

**FIG. 1**

Europäisches
Patentamt

European
Patent Office

Office européen
des brevets

## RAPPORT DE RECHERCHE EUROPEENNE

Numéro de la demande

EP 09 15 7300

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | US 2002/028260 A1 (WALKER EDWARD B [US] ET AL) 7 mars 2002 (2002-03-07) * alinéas [0017], [0033] - [0037], [0072]; revendications * ----- | 1-18 | INV. A23L1/29 A23L2/00 A61K36/45 A61P13/00 A61P31/04 |
| Y | WO 2008/031004 A (US GOVERNMENT [US]; DELEHANTY JAMES B [US]; WHITE BRANDY J [US]; LIN B) 13 mars 2008 (2008-03-13) * exemple 1 * ----- | 1-18 | |
| D,A | PORTER L J ET AL: "the conversion of procyanidins and prodelphinidins to cyanidin and delphinidin" PHYTOCHEMISTRY, PERGAMON PRESS, GB, vol. 25, no. 1, 1 janvier 1986 (1986-01-01), pages 223-230, XP002359377 ISSN: 0031-9422 * le document en entier * ----- | | |
| X | EP 1 632 486 A (KYOWA HAKKO KOGYO KK [JP]; NIKKA WHISKY DISTILLING CO LTD [JP] KYOWA H) 8 mars 2006 (2006-03-08) | 7-15 | DOMAINES TECHNIQUES RECHERCHES (IPC) A61K A61P |
| Y | * alinéas [0010] - [0014], [0019], [0021]; exemples 2,3 * ----- | 1-18 | |
| X | WO 99/12541 A (UNIV RUTGERS [US]) 18 mars 1999 (1999-03-18) * page 1, ligne 9 - ligne 17; revendications; exemple 1 * ----- | 1-18 | |

-/--

Le présent rapport a été établi pour toutes les revendications

| Lieu de la recherche | Date d'achèvement de la recherche | Examinateur |
|---|---|---|
| Munich | 24 juin 2009 | Langer, Astrid |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons
...........................................................................
& : membre de la même famille, document correspondant

EPO FORM 1503 03.82 (P04C02)

Europäisches
Patentamt

European
Patent Office

Office européen
des brevets

# RAPPORT DE RECHERCHE EUROPEENNE

Numéro de la demande

EP 09 15 7300

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | KRENN L ET AL: "Anthocyanin- and proanthocyanidin-rich extracts of berries in food supplements - analysis with problems"<br>PHARMAZIE,<br>vol. 62, no. 11, novembre 2007 (2007-11), pages 803-812, XP009108561<br>ISSN: 0031-7144<br>* le document en entier *<br>----- | 1-18 | |
| X | LAVIGNE J -P ET AL: "Cranberry (Vaccinium macrocarpon) and urinary tract infections: study model and review of literature"<br>PATHOLOGIE BIOLOGIE,<br>vol. 55, no. 8-9, novembre 2007 (2007-11), pages 460-464, XP022316821<br>ISSN: 0369-8114<br>* page 462, colonne 2, alinéa 5 - page 463, colonne 2, alinéa 1 *<br>----- | 1-3,17, 18 | |
| A | NOWACK R: "The American Cranberry (Vaccinium macrocarpon Aiton): Portrait of a herbal remedy"<br>ZEITSCHRIFT FUR PHYTOTHERAPIE 2003 DE,<br>vol. 24, no. 1, 2003, pages 40-46, XP009108541<br>ISSN: 0722-348X<br>* le document en entier *<br>----- | | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 24 juin 2009 | Langer, Astrid |

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 09 15 7300

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

24-06-2009

| Document brevet cité au rapport de recherche | | Date de publication | Membre(s) de la famille de brevet(s) | | Date de publication |
|---|---|---|---|---|---|
| US 2002028260 | A1 | 07-03-2002 | AUCUN | | |
| WO 2008031004 | A | 13-03-2008 | AUCUN | | |
| EP 1632486 | A | 08-03-2006 | EP | 1666476 A1 | 07-06-2006 |
| WO 9912541 | A | 18-03-1999 | AT | 413873 T | 15-11-2008 |
| | | | AU | 744527 B2 | 28-02-2002 |
| | | | AU | 9129698 A | 29-03-1999 |
| | | | CA | 2302743 A1 | 18-03-1999 |
| | | | DK | 1014969 T3 | 16-03-2009 |
| | | | EP | 1014969 A1 | 05-07-2000 |
| | | | ES | 2318874 T3 | 01-05-2009 |
| | | | JP | 2001515860 T | 25-09-2001 |
| | | | PL | 339144 A1 | 04-12-2000 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **Blumenthal M.** The ABC clinical guide to herbs. American Botanical Council, 2003, 73-83
- **Sobota A.E.** Inhibition of bacterial adherence by cranberry juice: potential use for the treatment of urinary tract infections. *J. Urol.,* 1984, vol. 131 (5), 1013-6
- **Howell A. B.** Inhibition of the adherence of P-fimbriated Escherichia coli to uroepithelial-cell surfaces by proanthocyanidins extracts from cranberries. *New Eng. J. Med.,* 1998, vol. 339 (15), 1085
- **Ofek L.** Anti-Escherichia coli adhesion activity of cranberry and blueberry juices. *New Eng. J. Med.,* 1991, vol. 324 (22), 1599
- **Stothers L.** A. randomised trial to evaluate effectiveness and cost effectiveness of naturopathic cranberry products as prophylaxis against urinary tract infection in women. *Can. J. Urol.,* 2002, vol. 9 (3), 1558-62
- **Bate-Smith E. C.** *Phytochemistry,* 1973, vol. 12, 907-912
- **Porter. L. J.** *Phytochemistry,* 1986, vol. 25, 223-230
- **Guyot et al.** *J.Agric.Food Chem.,* 1998, 1698-1705